# EUROPEAN PATENT SPECIFICATION

(11) **EP 4 085 859 B1**
(45) Date of publication and mention of the grant of the patent: **18.02.2026**
(21) Application number: 20910481.9
(22) Date of filing: 23.11.2020
(51) Int. Cl.: A61B 18/12, A61B 18/14, A61B 10/02

(54) **INTEGRATED ABLATION NEEDLE AND ABLATION SYSTEM**
INTEGRIERTE ABLATIONSNADEL UND ABLATIONSSYSTEM
AIGUILLE D'ABLATION INTÉGRÉE ET SYSTÈME D'ABLATION

(30) Priority: 31.12.2019 CN 201911424501; 31.12.2019 CN 201922501255 U
(43) Date of publication of application: 09.11.2022
(73) Proprietor: Hangzhou Nuo Cheng Medical Instrument Co., Ltd, Hangzhou, Zhejiang 310000 (CN)
(72) Inventor: QIU, Xinjiong, Hangzhou, Zhejiang 310000 (CN); WANG, Baidong, Hangzhou, Zhejiang 310000 (CN); ZHANG, Tingchao, Hangzhou, Zhejiang 310000 (CN); PENG, Bobo, Hangzhou, Zhejiang 310000 (CN); LIU, Liwen, Hangzhou, Zhejiang 310000 (CN)
(74) Representative: KIPA AB
(86) International application number: PCT/CN2020/130917
(87) International publication number: WO 2021/135726

(56) References cited:
- WO-A1-2009/156506
- WO-A1-2009/156506
- WO-A1-96/14018
- WO-A2-02/054957
- CN-A- 1 666 717
- CN-A- 103 876 785
- CN-A- 110 584 724
- CN-U- 203 789 956
- CN-U- 209 548 081
- CN-U- 209 548 081
- CN-U- 209 808 523
- CN-U- 209 808 524
- CN-U- 209 847 249
- CN-U- 211 934 280
- CN-U- 211 934 280
- US-A- 5 947 964
- US-A1- 2018 303 534
- US-B1- 6 485 436

## Description

### TECHNICAL FIELD

The present application relates to the technical field of medical devices, and in particular to an integrated ablation needle and an ablation system.

### BACKGROUND

With the development of minimally invasive medical technology, ablation has been widely used in the treatment of diseases such as liver, kidney and soft tissue tumors. In an ablation procedure, a radiofrequency ablation needle or microwave ablation needle is usually inserted into the lesion site to provide high temperature for the local tissue at the lesion site with radiofrequency energy or microwave energy to cause coagulation necrosis of the tissue, thereby treating the disease. In order to get the effect of the ablation treatment, it is necessary to take out the tissue sample for pathological analysis, i.e., biopsy, after the ablation is completed.

In the prior arts, the ablation needle and the biopsy needle are usually separated. The ablation needle usually first punctures into the diseased tissue to perform ablation, and after the ablation is completed, the ablation needle is withdrawn from the human body. After that, the biopsy needle then individually punctures into the ablated tissue to perform sampling. This means two punctures are required for the ablation and the biopsy after ablation. Puncturing repeatedly increases the damage to the human body's tissues and organs, increases the potential operative risk and extends the operation time. A biopsy needle known from International Publication Number WO2009/156506A1 includes two electric conductors for applying a voltage. The electric conductors are disposed and designed such that current can flow between the ends of the electric conductors. When obtaining a biopsy, the biopsy needle can be used to prevent tumor cell cross-contamination or bleeding. Chinese Utility Model Issue No. CN209548081U on which the preamble of claim 1 is based, discloses a multifunctional microwave thermal coagulation needle. The biopsy needle includes a puncture head, a needle tube, a handle and a biopsy tube, an insulator medium is arranged between the front end of the needle tube and the puncture head. International Publication Number WO02/054957A2 discloses a device to access a desired tissue site within a patient's body and separating a tissue specimen (biopsy) from the tissue site suitable for evaluation. The device includes a probe member having an arcuate tissue cutting RF powered electrode.

### SUMMARY

In view of the above, the present application provides an integrated ablation needle and an ablation system, in which the ablation and the biopsy are integrated on the same ablation needle, thereby avoiding puncturing repeatedly, reducing the damage to the human body, and saving the operation time.

In order to solve the above technical problems, the invention is set out in the appended set of claims. Preferable embodiments are defined in the dependent claims. In particular, the present application provides an integrated ablation needle, which comprises a sleeve and an electrode needle movably inserted in the sleeve. The electrode needle comprises a needle tip at a distal end and a needle bar connected to a proximal end of the needle tip. The needle bar has a sampling slot at least at a portion of the needle bar near the needle tip. A distal end of the sleeve is provided with a cutting portion. The sleeve is axially movable relative to the needle bar to expose or cover the sampling slot. When the sampling slot is exposed, tissue around the needle bar enters the sampling slot. When the sampling slot is covered, the cutting portion cuts the tissue inside the sampling slot off from the tissue outside the sampling slot to obtain the tissue in the sampling slot as a biopsy sample. The electrode needle is provided with a cooling channel extending axially from a proximal end face thereof to an interior of the needle tip, the cooling channel communicates with the sampling slot. An outer diameter of the needle bar is smaller than an inner diameter of the sleeve, and a proximal end of the sleeve and a proximal end of the electrode needle are both connected to a vacuum device.

The present application further provides an ablation system comprising the aforementioned integrated ablation needle and an energy generating device electrically connected to the electrode needle of the integrated ablation needle.

In the integrated ablation needle and the ablation system according to the present application, the integrated ablation needle comprises a sleeve and an electrode needle movably inserted into the sleeve, a sampling slot is opened on the needle bar of the electrode needle, and the distal end of the sleeve is provided with a cutting portion. The electrode needle is electrically connected to the energy generating device for performing ablation. After ablation, the sleeve is movable proximally to expose the sampling slot, so that tissue around the needle bar can enter the sampling slot, and the sleeve is movable distally to cover the sampling slot, so that the cutting portion of the sleeve at the distal end thereof can cut the tissue inside the sampling slot off from the tissue outside the sampling slot, thereby obtaining the tissue in the sampling slot as a biopsy sample. Therefore, that ablation and the biopsy are integrated on the integrated ablation needle, with no need for a separate biopsy operation, thereby avoiding puncturing repeatedly, reducing the damage to the human body, and saving the operation time.

### BRIEF DESCRIPTION OF THE DRAWINGS

In order to more clearly illustrate the technical solutions of the embodiments of the present application, the drawings accompanying the embodiments will be briefly described below, and it will be apparent that the drawings in the following description are some embodiments of the present application, and that, for those skilled in the art, other drawings can be obtained from the drawings without creative labor, as long as those drawings fall within the scope of the invention as claimed.
FIG. 1 is an overall perspective structural view of an integrated ablation needle according to a first embodiment of the present application.
FIG. 2 is an axial cross-sectional view of the sleeve of FIG. 1.
FIG. 3 is an enlarged view of part III of FIG. 2.
FIG. 4 is a schematic perspective view of the electrode needle of FIG. 1.
FIG. 5 is an axial cross-sectional view of the electrode needle of FIG. 4.
FIG. 6 is an axial cross-sectional view of the distal portion of the integrated ablation needle of FIG. 1, with the sampling slot exposed.
FIG. 7 is an axial cross-sectional view of the distal portion of the integrated ablation needle of FIG. 1, with the sampling slot covered.
FIG. 8 is an overall perspective structural view of an ablation system according to the present application.
FIGS. 9-11 are schematic views of a method for using the ablation system of FIG. 8.
FIG. 12 is a schematic partial view of an integrated ablation needle according to a second embodiment of the present application.
FIG. 13 is a schematic structural view of the distal portion of the sleeve of FIG. 12.
FIG. 14 is an axial cross-sectional partial view of an integrated ablation needle according to a third embodiment of the present application.
FIG. 15 is an axial cross-sectional view of the electrode needle of FIG. 14.
FIG. 16 is an enlarged schematic view of the XV portion of FIG. 14.

### DESCRIPTION OF THE EMBODIMENTS

The technical solutions according to the embodiments of the present application will be clearly and fully described below with reference to the drawings accompanying the embodiments of the present application. Apparently, the described embodiments are only part of, instead of all of the embodiments of the present application. All other embodiments falling within the scope of the invention as claimed fall into the protection scope of the present application.

In the description of the present application, it should be noted that terms denoting the orientation or positional relationships, such as "above", "below", "inner", "outer", refer to the orientation or positional relationships shown in the drawings for convenience of description, but not indicating or implying that the device or element referred to must have the particular orientation or be constructed and operated in the particular orientation, and thus cannot be construed as limiting the present application. In addition, terms such as "the first" and "the second" are used for descriptive purpose only and are not to be understood as indicating or implying relative importance.

In the description of the present application, it should be noted that in the field of interventional medical devices, the proximal end refers to the end closer to the operator, the distal end refers to the end far away from the operator, and the axial direction refers to the direction parallel to the line connecting the center of the distal end and the center of the proximal end of the medical device in the natural state. These terms are for convenience of description only and are not to be construed as limiting the present application.

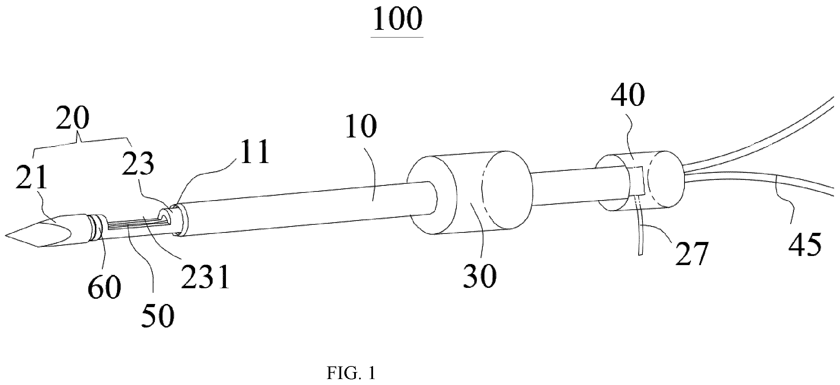

Referring to FIG. 1, the present application provides an integrated ablation needle 100 for performing ablation and obtaining biopsy sample after ablation. The integrated ablation needle 100 includes a sleeve 10 and an electrode needle 20 movably inserted into the sleeve 10. The electrode needle 20 includes a distal needle tip 21 and a needle bar 23 connected to a proximal end of the needle tip 21. The needle bar 23 has a sampling slot 231 at least near the needle tip 21. The sleeve 10 is provided with a cutting portion 11 at the distal end thereof. The sleeve 10 can be moved axially relative to the needle bar 23 to expose or cover the sampling slot 231. In the present application, the electrode needle 20 can be electrically connected with an energy generating device such as a radio frequency generator or a microwave generator for performing ablation. After ablation, the tissue around the needle bar 23 enters the sampling slot 231 when the sampling slot 231 is exposed, and the cutting portion 11 cuts off the tissue inside the sampling slot 231 from the tissue outside the sampling slot 231 when the sampling slot 231 is covered, thereby obtaining the tissue inside the sampling slot 231 as a biopsy sample. Therefore, the integrated ablation needle 100 integrates the ablation with the biopsy, with no need for a separate biopsy operation, thereby avoiding puncturing repeatedly, reducing the damage to the human body, and reducing the potential operative risk and saving the operation time.

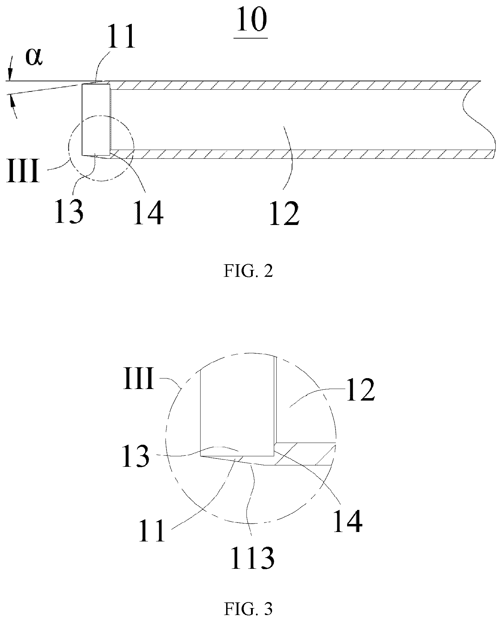

Specifically, referring to FIG. 2 and FIG. 3, the sleeve 10 is shaped as a hollowed circular tube, and has a lumen 12 extending axially and passing through two ends thereof so that the electrode needle 20 (see FIG. 1) can be movably inserted therein. The sleeve 10 has a circular receiving groove 13 in the distal inner wall thereof so that a first annular step 14 is formed at the junction of the receiving groove 13 and the lumen 12. The sleeve 10 further has the cutting portion 11 at the distal end thereof as described above, which includes a cutting surface 113 that is inclined with respect to the axis of the sleeve 10. The cutting surface 113 is closer to the axis of the sleeve 10 from its proximal end to its distal end. A first angle α is formed between the tangent line to the cutting surface 113 and the axis of the sleeve 10. To ensure the sharpness of the cutting surface 113, in this embodiment, the first angle α preferably ranges from 9 degrees to 17 degrees. Further, in this embodiment, the plane where the distal end of the cutting surface 113 is located is perpendicular to the axis of the sleeve 10, that is, the distal end of the sleeve 10 is a planar port.

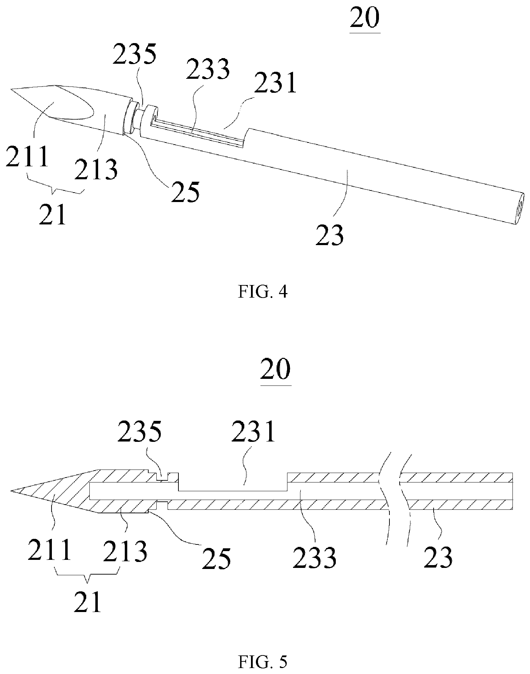

Referring to FIG. 4 and FIG. 5, the electrode needle 20 is substantially shaped as a round bar. The needle tip 21 includes a distal piercing section 211 and a connecting section 213 connected to a proximal end of the piercing section 211. The needle bar 23 is connected to a proximal end of the connecting section 213. Preferably, in this embodiment, the piercing section 211 is shaped as a sharp triangular pyramid or a tapered tip, so that the piercing section 211 has a good sharpness and thus can puncture the skin and the tissue more easily after the electrode needle 20 and the sleeve 10 are assembled. The connecting section 213 has a cylindrical shape, and the diameter of the connecting section 213 is larger than that of the needle bar 23, so that a second annular step 25 is formed at the junction of the connecting section 213 and the needle bar 23. Further, in the present application, in order to obtain the biopsy sample after ablation, the sampling slot 231 is at least defined at a portion of the needle bar 23 near the needle tip 21. Specifically, in this embodiment, the sampling slot 231 is a long narrow groove extending along the axial direction of the needle bar 23. The cross section of the sampling slot 231 perpendicular to the axial direction of the needle bar 23 is semicircular, that is, the sampling slot 231 is formed by axially removing half of the material of the needle bar 23 near the needle tip 21. The sampling slot 231 has a large sampling space, so that the tissue around the needle bar 23 can enter the sampling slot 231 more easily. Alternatively, in other embodiments, the cross section of the sampling slot 231 perpendicular to the axial direction of the needle bar 23 can be shaped as a sector, a rectangle, or the like, provided that the tissue around the needle bar 23 can be received therein.

In other embodiments, the sampling slot 231 can include a plurality of long narrow grooves extending along the axial direction of the needle bar 23, and the plurality of long narrow grooves can be spaced apart from each other in the circumferential or axial direction of the needle bar 23.

In other embodiments, the sampling slot 231 can include one or a plurality of arc grooves extending along the circumferential direction of the needle bar 23, and the plurality of arc grooves are spaced apart from each other in the circumferential or axial direction of the needle bar 23.

In other embodiments, the sampling slot 231 can include one or a plurality of annular grooves extending one round along the circumferential direction of the needle bar 23, and the plurality of annular grooves are spaced apart from each other in the axial direction of the needle bar 23.

Referring to FIG. 1, the integrated ablation needle 100 further includes a handle 30 connected to the proximal end of the sleeve 10 and the proximal end of the electrode needle 20. The handle 30 is configured to move the sleeve 10 axially relative to the needle bar 23, thereby exposing or covering the sampling slot 231.

Specifically, as shown in FIG. 1, the proximal end of the sleeve 10 is fixedly connected with a driving mechanism inside the handle 30 by over-molding or adhesive bonding, and the proximal end of the sleeve 10 extends out of the proximal end of the handle 30. The needle tip 21 of the electrode needle 20 is located outside the sleeve 10, and the needle bar 23 of the electrode needle 20 is movably inserted in the sleeve 10. The proximal end of the needle bar 23 passes through the proximal end of the handle 30 within the sleeve 10, and extends out of the proximal end of the sleeve 10. The proximal end of the needle bar 23 is externally connected to the energy generating device (not shown) such as the radio frequency generator or the microwave generator through an electrode wire 27, so that the needle tip 21 at the distal end of the electrode needle 20 outside the sleeve 10 can be used for ablation. Specifically, in the case where the electrode needle 20 is electrically connected to the radio frequency generator, the electrode needle 20 transmits a high frequency current to cause the charged positive and negative ions in the diseased tissue around the distal end of the electrode needle 20 to oscillate at high speed. The ions oscillating at high speed generate a large amount of heat by friction, thereby raising the temperature of the diseased tissue. Finally, the protein in the diseased cells is denatured, the intracellular water and extracellular water are lost, and the diseased tissue is coagulated and necrotic, so as to achieve radiofrequency ablation. In the case where the electrode needle 20 is electrically connected to the microwave generator, the distal end of the electrode needle 20 forms a microwave field, dipole molecules such as water molecules in the diseased tissue generate heat by motion friction and violent collision under the action of the microwave field, thereby raising the temperature of the diseased tissue. Finally, the protein in the diseased cells is denatured, the intracellular water and extracellular water are lost, and the diseased tissue is coagulated and necrotic, so as to achieve microwave ablation. In this embodiment, the electrode needle 20 is preferably made of a biocompatible metal having excellent electrical conductivity such as stainless steel, and the proximal end of the needle bar 23 is electrically connected to the radio frequency generator through the electrode wire 27. That is, the needle tip 21 is used for radiofrequency ablation.

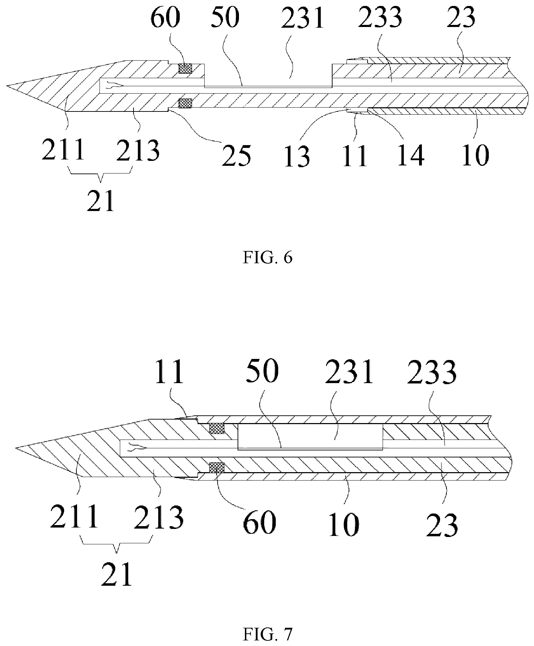

As described above, the proximal end of the sleeve 10 is fixedly connected with the driving mechanism in the handle 30, and the needle bar 23 is movably received in the sleeve 10, so that the sleeve 10 can be moved relative to the needle bar 23 in the axial direction by moving the driving mechanism in the handle 30 in the axial direction, thereby exposing or covering the sampling slot 231 in the needle bar 23 as required. Specifically, in some embodiments, as the driving mechanism within the handle 30 is moved proximally a certain distance, the handle 30 moves the sleeve 10 proximally until the sampling slot 231 is exposed, as shown in FIG. 6. In some embodiments, as the driving mechanism in the handle 30 is moved distally a certain distance, the handle 30 moves the sleeve 10 distally until the sampling slot 231 is covered, as shown in FIG. 7.

Optionally, the sleeve 10 is provided with a first positioning portion, and the electrode needle 20 is provided with a second positioning portion. The first positioning portion is configured to be engaged with the second positioning portion to achieve the axial positioning between the electrode needle 20 and the sleeve 10, thereby indicating whether the sampling slot 231 is completely covered, so that the situation that the sampling slot 231 is not completely covered and thus the tissue inside the sampling slot 231 is not completely cut off from the tissue outside the sampling slot 231 can be avoided.

Specifically, as shown in FIG. 7, in this embodiment, the diameter of the lumen 12 of the sleeve 10 is larger than the diameter of the needle bar 23 and smaller than the diameter of the needle tip 21, and the diameter of the receiving groove 13 of the sleeve 10 is larger than the diameter of the needle tip 21, so that the first annular step 14 on the sleeve 10 can abut against the second annular step 25 on the electrode needle 20 by moving the sleeve 10 distally relative to the needle bar 23 through the handle 30, thereby achieving the axial positioning between the electrode needle 20 and the sleeve 10, in which the connecting section 213 of the proximal end of the needle tip 21 is partially received in the receiving groove 13 of the sleeve 10, and the sampling slot 231 of the needle bar 23 is completely covered. It can be seen that the first annular step 14 functions as the first positioning portion, and the second annular step 25 functions as the second positioning portion.

In other embodiments, the first positioning portion and the second positioning portion can be a first developing point and a second developing point, respectively, which can be developed under a medical imaging device. The first developing point is provided on the outer peripheral surface or the inner peripheral surface of the sleeve 10 at the distal end thereof close to the cutting portion 11 of the sleeve 10 at the distal end thereof. The second developing point is provided on the outer peripheral surface of the connecting section 213 of the needle tip 21 close to the sampling slot 213. The operator determines whether or not the sampling slot 231 is completely covered by observing the relative position between the first developing point and the second developing point under the medical imaging device. When the first developing point and the second developing point coincide or partially coincide, it can be determined that the sampling slot 231 is completely covered. Furthermore, the first developing point and the second developing point can help the operator to determine whether the distal end of the electrode needle 20 reaches or is located at a predetermined ablation location.

The first developing point and the second developing point can be developed by, for example, ultrasonic development or X-ray fluoroscopy, and preferably by ultrasonic development which is less harmful to human body, with lower cost.

It can be understood that, by driving the sleeve 10 to move axially relative to the needle bar 23 through the driving mechanism in the handle 30, not only the sampling slot 231 can be exposed or covered, the length of the distal end of the electrode needle 20 extending out of the sleeve 10 can also be adjusted, thereby changing the effective ablation length of the integrated ablation needle 100 to meet the ablation requirements at different lesion sites or different patients.

The sleeve 10 surrounding the electrode needle 20 is at least partially insulated, that is, the sleeve 10 can be completely insulated or partially insulated. In the case where the sleeve 10 is completely insulated, the portion of the electrode needle 20 at the distal end thereof extending out of the sleeve 10 performs ablation, and the length of the portion of the electrode needle 20 at the distal end thereof extending out of the sleeve 10 is the effective ablation length of the integrated ablation needle 100. In the case where the sleeve 10 is partially insulated, the portion of the electrode needle 20 at the distal end thereof extending out of the sleeve 10 and the non-insulated portion of the sleeve 10 perform ablation, the effective ablation length of the integrated ablation needle 100 is the sum of the length of the portion of the electrode needle 20 at the distal end thereof extending out of the sleeve 10 and the length of the non-insulated portion of the sleeve 10. Preferably, in this embodiment, the sleeve 10 is completely insulated. The entirety of the sleeve 10 can be made of an insulating material, for example, a plastic tube such as PEEL, PI or PA that meets the hardness requirements, or a ceramic tube such as alumina porcelain, steatite porcelain, or boron nitride. The entirely of the sleeve 10 can be made of a non-insulated material, and the outer surface of the sleeve 10 is covered with an insulating coating. In this embodiment, in order to improve the rigidity of the sleeve 10 and facilitate the puncturing of the sleeve 10 into human tissue, preferably, the sleeve 10 is made of a metal material, and the outer surface of the tube of the sleeve 10 is coated with an insulating coating. The metal material includes, but is not limited to, 304 stainless steel, 321stainless steel, or 631 stainless steel. The insulating coating includes, but is not limited to, PTFE coating, titanium nitride coating, parylene coating, or the like. The metal material of which the sleeve 10 is made should have sufficient hardness to puncture into human tissue, as well as excellent biocompatibility. The insulating coating should have reliable insulation and excellent biocompatibility, with a small coefficient of friction. Further, the insulating coating needs to be closely bonded with the outer surface of the tube of the sleeve 10, and is not easy to peel off. For example, a 304 stainless steel tube with PTFE coating, a 304 stainless steel tube with parylene coating, a 321 stainless steel tube with titanium nitride coating, or a 631 stainless steel tube with parylene coating can be used. Considering the insulation reliability and the process feasibility, the thickness of any kind of the insulating coatings should be no smaller than 3 µm.

It can be conceived that, in other embodiments, the sleeve 10 can be partially insulated, and the non-insulated portion of the sleeve 10 can be used to transmit high frequency current or microwave so as to enlarge the ablation area.

Referring to FIG. 1 and FIG. 7, as described above, after the electrode needle 20 is electrically connected to the radio frequency generator or the microwave generator, the portion of the electrode needle 20 contacting the tissue will transmit the radio frequency energy or the microwave energy to cause the high temperature of the local tissue at the lesion, so that the tissue at the lesion can be coagulated and necrotized, thereby treating the disease. However, an excessively high, local temperature will affect the normal tissue that does not need to be ablated. Therefore, in this application, a cooling channel 233 is defined in the electrode needle 20 for delivering a cooling medium, such as cooling water, in a gaseous or liquid state for cooling to control the temperature during the ablation.

Specifically, as shown in FIG. 1, the cooling channel 233 is a cylindrical channel, and the cooling channel 233 extends from the proximal end face of the needle bar 23 to the interior of the needle tip 21. The proximal end of the sleeve 10 and the proximal end of the electrode needle 20 are connected to a vacuum device 40, so that the vacuum device 40 communicates with the cooling channel 233. A pair of circulating tubes 45 is disposed at the proximal end of the vacuum device 40. The pair of circulating tubes 45 is externally connected with a cooling medium supplying device (not shown) to provide sufficient cooling medium to ensure that the ablation temperature is maintained at a relatively ideal level. Optionally, a temperature measuring element 50 extending into the interior of the needle tip 21 is provided in the cooling channel 233 to obtain the ablation temperature in real time. In this embodiment, the temperature measuring element 50 is a temperature measuring wire extending into the interior of the needle tip 21.

As shown in FIG. 7, the cooling channel 233 communicates with the sampling slot 231, so that when the sleeve 10 covers the sampling slot 231 and the electrode needle 20 performs ablation, the cooling medium can flow between the sleeve 10 and the electrode needle 20 through the sampling slot 231, so that the cooling can be more uniform. Preferably, in this embodiment, the diameter of the needle bar 23 is smaller than the diameter of the lumen 12 of the sleeve 10, so that a liquid space other than the cooling channel 233 can be provided for the cooling medium, and the cooling can be faster.

Further, in order to prevent the leakage of the cooling medium from the radial gap between the sleeve 10 and the needle bar 23 during ablation, a sealing mechanism is provided on the sleeve 10 and / or the needle bar 23 to seal the radial gap between the sleeve 10 and the needle bar 23, and when the sampling slot 231 is closed by the sleeve 10, the sealing mechanism is located between the sampling slot 231 and the needle tip 21. Specifically, referring to FIGS. 5 and 7, in this embodiment, the needle bar 23 provides a circular sealing groove 235 near the needle tip 21, and the sealing groove 235 is located between the sampling slot 231 and the needle tip 21. A sealing ring 60 is received in the sealing groove 235, and the sealing groove 235 and the sealing ring 60 form the sealing mechanism. The sealing ring 60 has elasticity and the outer diameter of the sealing ring 60 is slightly larger than the diameter of the lumen 12 of the sleeve 10, so that after the electrode needle 20 is engaged with the sleeve 10, the sealing ring 60 is elastically deformed so as to closely contact the inner peripheral surface of the sleeve 10, with a good sealing effect.

In other embodiments, a plurality of sealing grooves 235 can be axially defined in a portion of the needle bar 23 adjacent to the needle tip 21, and each of the sealing grooves 235 is provided with a sealing ring 60 to enhance the sealing effect.

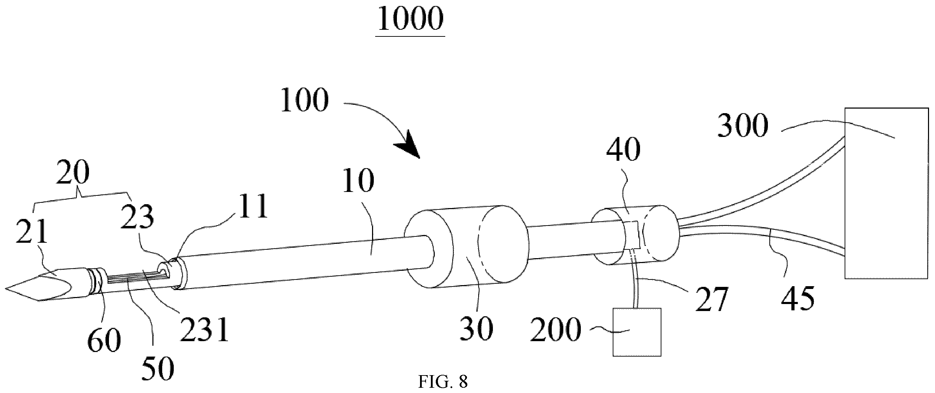

Referring to FIG. 8, the present application further provides an ablation system 1000, including the integrated ablation needle 100 and the energy generating device 200 electrically connected to the electrode needle 20 of the integrated ablation needle 100. The energy generating device 200 includes, but is not limited to, a radio frequency generator or a microwave generator. In this embodiment, the energy generating device 200 is a radio frequency generator. The radio frequency generator is electrically connected to the electrode needle 20 through the electrode wire 27 for supplying high frequency current to the electrode needle 20 for radio frequency ablation.

The ablation system 1000 further includes the cooling medium supplying device 300 that communicates with the vacuum device 40 of the integrated ablation needle 100 through a pair of circulating tubes 45 and thus communicates with the cooling channel 233 of the electrode needle 20 to provide sufficient cooling medium in a gaseous or liquid state into the cooling channel 233 for controlling the temperature of the integrated ablation needle 100 during ablation.

Referring to FIG. 1, FIG. 9 and FIG. 11, an illustrative method for using the ablation system 100, the method not falling under the scope of the invention as claimed, is described as follows:

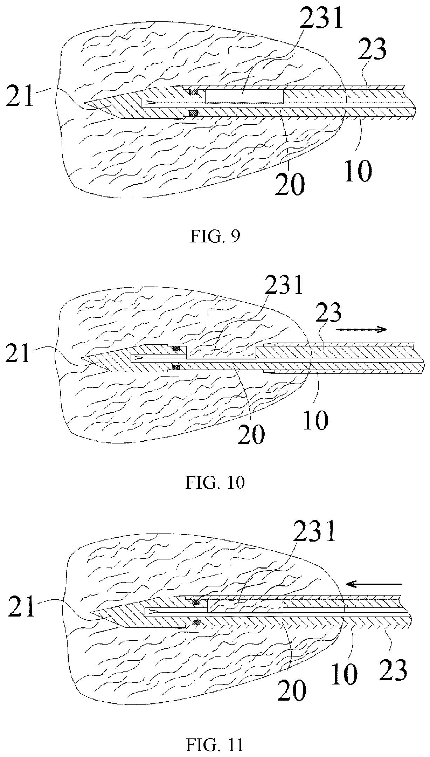

In the first step, as shown in FIG. 9, the electrode needle 20 is inserted into the sleeve 10, the electrode needle 20 and the sleeve 10 are in a closed state, and the sleeve 10 completely covers the sampling slot 231 in the needle bar 23. Due to the sealing ring, a complete sealing between the sleeve 10 and the electrode needle 20 is ensured. Under the guidance of ultrasonic development, the sleeve 10 and the electrode needle 20 in the closed state reach the lesion site of the human body through the puncture of the needle tip 21. A high-frequency current is supplied to the electrode needle 20 through the energy generating device 200 (see FIG. 8). The distal needle tip 21 of the electrode needle 20 discharges to perform radiofrequency ablation on the lesion site, the temperature measuring element 50 (see FIG. 7) monitors the ablation temperature in real time, and the cooling medium supplying device 300 (see FIG. 8) introduces the cooling medium into the cooling channel 233 of the electrode needle 20 to realize circulating cooling.

In the second step, after the ablation is completed, the cooling medium supplying device 300 is closed, the vacuum device 40 is opened, and the cooling medium is completely sucked out by negative pressure effect. After the cooling medium is completely discharged by the vacuum device 40, the handle 30 is operated to drive the sleeve 10 connected therewith to move proximally relative to the needle bar 23 of the electrode needle 20 to completely or partially expose the sampling slot 231. The ablated tissue around the needle bar 23 will partially enter the sampling slot 231 and the vacuum device 40, which is still working, will provide a negative pressure on the tissue, causing the tissue to be drawn into the sampling slot 231, as shown in FIG. 10.

In the third step, the handle 30 is operated to drive the sleeve 10 to move distally relative to the needle bar 23 of the electrode needle 20, until the sampling slot 231 is completely covered. The tissue inside the sampling slot 231 is cut off from the tissue outside the sampling slot 231 by the cutting portion 11 of the sleeve 10 at the distal end thereof, so that the tissue within the sampling slot 231 is separated from other tissue and retained within the sampling slot 231, as shown in FIG. 11. Finally, the entire integrated ablation needle 100 is withdrawn from the human body, and the tissue in the sampling slot 231 is taken out for test and analysis as a biopsy sample.

The integrated ablation needle 100 and the ablation system 1000 according to the present application integrate the ablation and the biopsy on the same ablation needle, with no need to perform a separate biopsy step, thereby avoiding puncturing repeatedly, reducing the damage to the human body, and reducing the potential operative risk and saving the operation time.

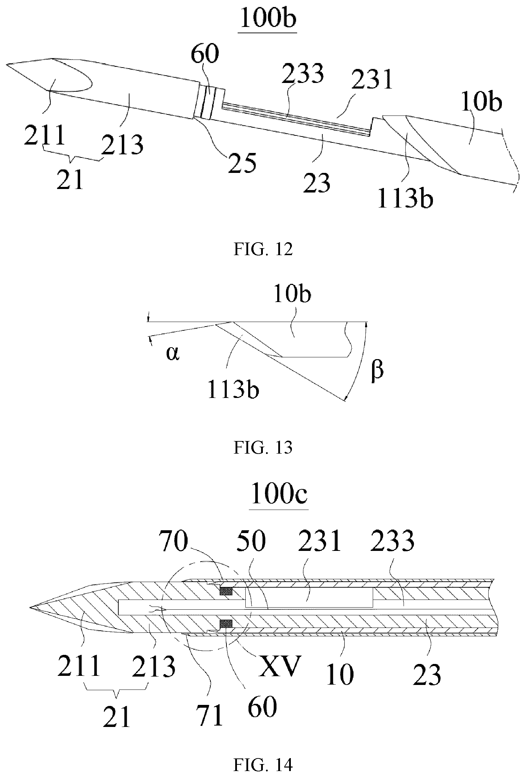

Referring to FIGS. 12 and 13, the structure of the integrated ablation needle 100b according to the second embodiment of the present application is similar to the structure of the integrated ablation needle 100 according to the first embodiment, except that in the second embodiment, a second angle β is formed between the plane where the distal end of the cutting surface 113b is located and the axis of the sleeve 10b. That is, the distal end of the sleeve 10b is a chamfered tip. Compared with the planar port, the distal end of the cutting surface 113b has a smaller contact area with the tissue when the chamfered tip according the present embodiment cuts the tissue, and with the same driving force, the cutting surface 113b with smaller contact area can cut the tissue more easily.

In order to ensure the sharpness of the cutting surface 113b during cutting, in this embodiment, the second angle β preferably ranges from 25 degrees to 35 degrees.

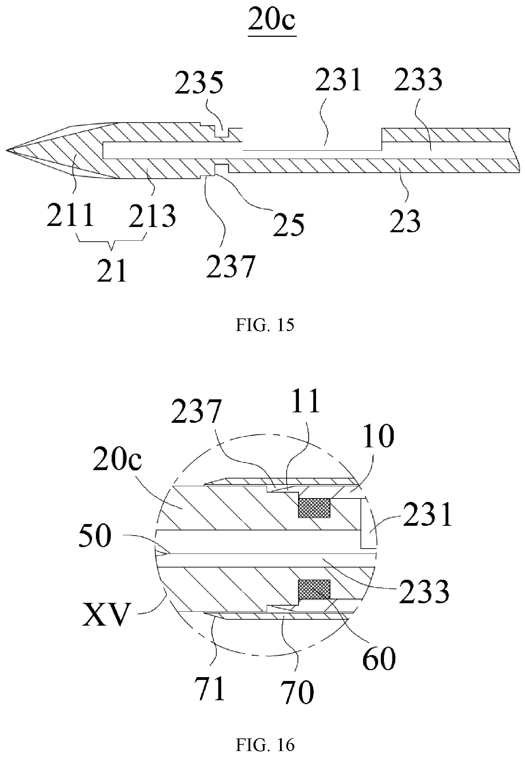

Referring to FIGS. 14 to 16, the structure of the integrated ablation needle 100c according to the third embodiment of the present application is similar to the structure of the integrated ablation needle 100 according to the first embodiment, except that in the third embodiment, the integrated ablation needle 100c further includes an outer tube 70 surrounding the sleeve 10, and the electrode needle 20 and the sleeve 10 are axially movable relative to the outer tube 70, so that the integrated ablation needle 100c can perform pre-ablation biopsy, ablation, and post-ablation biopsy with one puncture.

Specifically, in use of the integrated ablation needle 100c, the outer tube 70 surrounds the sleeve 10 and is punctured into the diseased tissue together with the sleeve 10 and the electrode needle 20. Prior to ablation, the outer tube 70 is moved axially proximally relative to the sleeve 10 and the electrode needle 20 to expose the distal portions of the sleeve 10 and the electrode needle 20 that are assembled together; and after that, the sleeve 10 is moved axially relative to the electrode needle 20 to obtain a pre-ablation tissue sample, the sleeve 10 and the electrode needle 20 assembled together are then withdrawn outside of the human body from the outer tube 70, with the outer tube 70 remained in the tissue, and the pre-ablation tissue sample in the sampling slot 231 of the electrode needle 20 is taken out. After that, the sleeve 10 and the electrode needle 20 assembled together enter the lesion site of the diseased tissue via the outer tube 70 retained in the tissue as a passage to perform ablation. After ablation, the sleeve 10 is moved axially relative to the electrode needle 20 again to obtain the ablated tissue sample. Finally, the outer tube 70, the sleeve 10 and the electrode needle 20 are withdrawn from the human body together.

In order to avoid excessive resistance during puncture due to an excessively large outer diameter of the integrated ablation needle 100c due to the outer tube 70, in this embodiment, the proximal end of the needle tip 21 is provided with an avoidance groove 237 around the circumference thereof. The second annular step 25 is formed at the junction of the avoidance groove 237 and the sealing groove 235 in the needle bar 23. When the sleeve 10 is axially positioned with respect to the electrode needle 20, the first annular step 14 (not shown) of the sleeve 10 abuts against the second annular step 25, and the cutting portion 11 of the sleeve 10 is received in the avoidance groove 237. The outer diameter of the sleeve 10 is equal to or smaller than the diameter of the proximal end of the needle tip 21, so that the outer diameter of the integrated ablation needle 100c does not increase after the outer tube 70 surrounds the sleeve 10, which facilitates the puncturing. In addition, the cutting portion 11 of the sleeve 10 is received in the avoidance groove 237, so that the cutting portion 11 of the sleeve 10 can be protected.

Preferably, the distal end of the outer tube 70 is provided with an inclined surface 71 which is closer to the axis of the outer tube 70 from the proximal end to the distal end thereof for reducing the resistance to the outer tube 70 entering the tissue, which facilitates the puncturing.

In this embodiment, the outer tube 70 is added to the outside of the sleeve 10 and can be inserted into the tissue together with the sleeve 10 and the electrode needle 20, and then the outer tube 70 can be retained in the tissue as a passage for pre-ablation biopsy, ablation and post-ablation biopsy, thereby avoiding puncturing repeatedly, reducing the damage to the human body, and reducing the potential operative risk and saving the operation time.

It should be noted that modifications and developments made to the above embodiment of the present application by those skilled in the art without departing from the scope of the invention as claimed fall in the protection scope of this application.

## Claims

1. An integrated ablation needle (100, 100b, 100c), comprising a sleeve (10) and an electrode needle (20) movably inserted in the sleeve (10), wherein the electrode needle (20) comprises a needle tip (21) at a distal end thereof and a needle bar (23) connected with a proximal end of the needle tip (21), and wherein a sampling slot (231) is opened at least at a portion of the needle bar (23) adjacent to the needle tip (21), and wherein a cutting portion (11) is provided at a distal end of the sleeve (10); and
wherein the sleeve (10) is movable axially relative to the needle bar (23) to expose or cover the sampling slot (231); and wherein when the sampling slot (231) is exposed, tissue around the needle bar (23) enters the sampling slot (231), and when the sampling slot (231) is covered, the cutting portion (11) cuts the tissue inside the sampling slot (231) off from the tissue outside the sampling slot (231), thereby obtaining the tissue in the sampling slot (231) as a biopsy sample,
wherein the electrode needle (20) is provided with a cooling channel (233) extending axially from a proximal end face thereof to an interior of the needle tip (21), and an outer diameter of the needle bar (23) is smaller than an inner diameter of the sleeve (20), and
**characterized in that**
the cooling channel (233) communicates with the sampling slot (231) and
a proximal end of the sleeve (20) and a proximal end of the electrode needle (20) are both connected to a vacuum device (40).

2. The integrated ablation needle (100, 100b, 100c) according to claim 1, wherein the sleeve (10) is at least partially insulated.

3. The integrated ablation needle (100, 100b, 100c) according to claim 1, wherein the sampling slot (231) includes a plurality of long narrow grooves extending along an axial direction of the needle bar, and wherein the plurality of long narrow grooves are spaced apart from each other along a circumferential or axial direction of the needle bar; or the sampling slot (231) includes a plurality of arc grooves extending in a circumferential direction of the needle bar, and wherein the plurality of arc grooves are spaced apart from each other along the circumferential or an axial direction of the needle bar (23); or the sampling slot (231) includes a plurality of annular grooves extending one round in a circumferential direction of the needle bar (23), and wherein the plurality of annular grooves are spaced apart from each other along an axial direction of the needle bar (23).

4. The integrated ablation needle (100, 100b, 100c) according to claim 1, wherein the cutting portion (11) comprises a cutting surface (113) inclined with respect to an axis of the sleeve (10), the cutting surface (113) is closer to the axis of the sleeve (10) from a proximal end to a distal end thereof, a first angle (α) is formed between a tangent line of the cutting surface (113) and the axis of the sleeve (10), and the first angle (α) ranges from 9 degrees to 17 degrees.

5. The integrated ablation needle (100, 100b, 100c) according to claim 4, wherein a plane where the distal end of the cutting surface (113) is located is perpendicular to the axis of the sleeve (10); or a second angle (β) is formed between the plane where the distal end of the cutting surface (113b) is located and the axis of the sleeve (10b), wherein the second angle (β) ranges from 25 degrees to 35 degrees.

6. The integrated ablation needle (100, 100b, 100c) according to claim 1, wherein the sleeve (10) is provided with a first positioning portion, the electrode needle (20) is provided with a second positioning portion, wherein the first positioning portion and the second positioning portion is engageable to each other to achieve axial positioning between the electrode needle and the sleeve.

7. The integrated ablation needle (100, 100b, 100c) according to claim 6, wherein the sleeve (10) has a circular receiving groove (13) in a distal inner wall thereof, and a first annular step (14) is formed at a junction between a lumen of the sleeve (10) and the receiving groove (13) as the first positioning portion; a diameter of the proximal end of the needle tip (21) is larger than a diameter of the needle bar (23), and a second annular step (25) is formed at a junction between the proximal end of the needle tip (21) and the needle bar (23) as the second positioning portion; and when the first annular step (14) abuts the second annular step (25), the electrode needle (20) and the sleeve (10) are axially positioned, and a proximal portion of the needle tip (21) is received in the receiving groove (13).

8. The integrated ablation needle (100, 100b, 100c) according to claim 1, wherein a sealing mechanism is provided on the sleeve (10) and / or the needle bar (23) to seal a radial gap between the sleeve (10) and the needle bar (23), and the sealing mechanism is located between the sampling slot (231) and the needle tip (21) when the sampling slot (231) is covered by the sleeve (10).

9. The integrated ablation needle (100, 100b, 100c) according to claim 8, wherein the needle bar (23) is provided with at least one circular sealing groove (235) adjacent to the needle tip (21), the sealing groove (235) is located between the sampling slot (231) and the needle tip (21), and a sealing ring (60) is received in the sealing groove (235), wherein the sealing groove (235) and the sealing ring (60) forms the sealing mechanism.

10. The integrated ablation needle (100, 100b, 100c) according to claim 1, further comprising a handle (30) connected to a proximal end of the sleeve (10) and a proximal end of the electrode needle (20), the handle (30) is used to drive the sleeve (10) to move axially relative to the needle bar (23).

11. The integrated ablation needle (100, 100b, 100c) according to any one of claims 1 to 10, further comprising an outer tube (70) surrounding the sleeve (10), and the electrode needle (20) and the sleeve (10) are axially movable relative to the outer tube (70).

12. The integrated ablation needle (100, 100b, 100c) according to claim 11, wherein the proximal end of the needle tip (21) is provided with an avoidance groove (237) around a circumference thereof, and when the sleeve (10) is axially positioned relative to the electrode needle (20), the cutting portion (11) of the sleeve (10) is received in the avoidance groove (237), and an outer diameter of the sleeve (10) is equal to or smaller than a diameter of the proximal end of the needle tip (21).

13. The integrated ablation needle (100, 100b, 100c) according to claim 1, wherein the sampling slot (231) is a long narrow groove extending along an axial direction of the needle bar; or the sampling slot (231) is an arc groove extending in a circumferential direction of the needle bar; or the sampling slot (231) is an annular groove extending one round in a circumferential direction of the needle bar (23).

## Patentansprüche

1. Eine integrierte Ablationsnadel (100, 100b, 100c), umfassend eine Hülse (10) und eine in die Hülse (10) beweglich eingeführte Elektrodennadel (20), wobei die Elektrodennadel (20) eine Nadelspitze (21) an ihrem distalen Ende und einen mit dem proximalen Ende der Nadelspitze verbundenen Nadelschaft (23) umfasst; und wobei ein Probenentnahmeschlitz (231) zumindest an einem Abschnitt des Nadelschafts (23) neben der Nadelspitze (21) geöffnet ist; und wobei ein Schneidabschnitt (11) an einem distalen Ende der Hülse (10) vorgesehen ist; und
wobei die Hülse (10) relativ zum Nadelschaft (23) axial beweglich ist, um den Probenentnahmeschlitz (231) freizulegen oder abzudecken; und wobei, wenn der Probenentnahmeschlitz (231) freigelegt ist, Gewebe um den Nadelschaft (23) in den Probenentnahmeschlitz (231) gelangt; und wobei, wenn der Probenentnahmeschlitz (231) abgedeckt ist, der Schneidabschnitt (11) das Gewebe innerhalb des Probenentnahmeschlitzes (231) vom Gewebe außerhalb des Probenentnahmeschlitzes (231) abschneidet, wodurch das Gewebe im Probenentnahmeschlitz (231) als Biopsieprobe gewonnen wird;
wobei die Elektrodennadel (20) mit einem Kühlkanal (233) versehen ist, der sich axial von einer proximalen Endfläche derselben zu einem Inneren der Nadelspitze (21) erstreckt; und wobei ein Außendurchmesser des Nadelschafts (23) kleiner ist als ein Innendurchmesser der Hülse (20);
**dadurch gekennzeichnet, dass**
der Kühlkanal (233) mit dem Probenentnahmeschlitz (231) in Verbindung steht und ein proximales Ende der Hülse (20) und ein proximales Ende der Elektrodennadel (20) beide mit einer Vakuumvorrichtung (40) verbunden sind.

2. Die integrierte Ablationsnadel (100, 100b, 100c) nach Anspruch 1, wobei die Hülse (10) zumindest teilweise isoliert ist.

3. Die integrierte Ablationsnadel (100, 100b, 100c) nach Anspruch 1, wobei der Probenentnahmeschlitz (231) mehrere lange, schmale Nuten umfasst, die sich entlang einer axialen Richtung des Nadelschafts erstrecken; und wobei die mehreren langen, schmalen Nuten entlang einer Umfangs- oder axialen Richtung des Nadelschafts voneinander beabstandet sind; oder der Probenentnahmeschlitz (231) eine Vielzahl von bogenförmigen Nuten umfasst, die sich in einer Umfangsrichtung des Nadelschafts erstrecken; und wobei die Vielzahl von bogenförmigen Nuten entlang der Umfangs- oder einer Axialrichtung des Nadelschafts (23) voneinander beabstandet sind; oder der Probeentnahmeschlitz (231) eine Vielzahl von ringförmigen Nuten umfasst, die sich einmal in Umfangsrichtung des Nadelschafts (23) erstrecken, wobei die Vielzahl von ringförmigen Nuten entlang einer axialen Richtung des Nadelschafts (23) voneinander beabstandet sind.

4. Die integrierte Ablationsnadel (100, 100b, 100c) nach Anspruch 1, wobei der Schneidabschnitt (11) eine Schneidfläche (113) umfasst, die in Bezug auf eine Achse der Hülsen (10) geneigt ist, die Schneidfläche (113) von einem proximalen Ende zu einem distalen Ende hin näher an der Achse der Hülse (10) liegt, ein erster Winkel (α) zwischen einer Tangente der Schneidfläche (113) und der Achse der Hülse (10) gebildet wird und der erste Winkel (α) im Bereich von 9 bis 17 Grad liegt.

5. Die integrierte Ablationsnadel (100, 100b, 100c) nach Anspruch 4, wobei eine Ebene, in der sich das distale Ende der Schneidfläche (113) befindet, senkrecht zur Achse der Hülse (10) steht; oder ein zweiter Winkel (β) zwischen der Ebene, in der sich das distale Ende der Schneidfläche (113b) befindet, und der Achse der Hülse (10b) gebildet wird, wobei der zweite Winkel (β) zwischen 25 und 35 Grad liegt.

6. Die integrierte Ablationsnadel (100, 100b, 100c) nach Anspruch 1, wobei die Hülse (10) mit einem ersten Positionierungsabschnitt versehen ist, die Elektrodennadel (20) mit einem zweiten Positionierungsabschnitt versehen ist und wobei der erste Positionierungsabschnitt und der zweite Positionierungsabschnitt miteinander in Eingriff gebracht werden können, um eine axiale Positionierung zwischen der Elektrodennadel und der Hülse zu erreichen.

7. Die integrierte Ablationsnadel (100, 100b, 100c) nach Anspruch 6, wobei die Hülse (10) eine kreisförmige Aufnahmenut (13) in ihrer distalen Innenwand aufweist und eine erste ringförmige Stufe (14) an einer Verbindungsstelle zwischen einem Lumen der Hülse (10) und der Aufnahmenut (13) als erster Positionierungsabschnitt ausgebildet ist; der Durchmesser des proximalen Endes der Nadelspitze (21) größer ist als der Durchmesser des Nadelschafts (23), und eine zweite ringförmige Stufe (25) an einer Verbindungsstelle zwischen dem proximalen Ende der Nadelspitze (21) und dem Nadelschaft (23) als zweiter Positionierungsabschnitt ausgebildet ist; und wenn die erste ringförmige Stufe (14) an die zweite ringförmige Stufe (25) stößt, die Elektrodennadel (20) und die Hülse (10) axial positioniert werden und ein proximaler Abschnitt der Nadelspitze (21) in der Aufnahmenut (13) aufgenommen wird.

8. Die integrierte Ablationsnadel (100, 100b, 100c) nach Anspruch 1, wobei ein Dichtungsmechanismus an der Hülse (10) und/oder dem Nadelschaft (23) vorgesehen ist, um einen radialen Spalt zwischen der Hülse (10) und dem Nadelschaft (23) abzudichten, und der Dichtungsmechanismus sich zwischen dem Probenentnahmeschlitz (231) und der Nadelspitze (21) befindet, wenn der Probenentnahmeschlitz (231) von der Hülse (10) abgedeckt ist.

9. Die integrierte Ablationsnadel (100, 100b, 100c) nach Anspruch 8, wobei der Nadelschaft (23) mit mindestens einer kreisförmigen Dichtungsnut (235) neben der Nadelspitze (21) versehen ist, die Dichtungsnut (235) zwischen dem Probeentnahmeschlitz (231) und der Nadelspitze (21) angeordnet ist und ein Dichtungsring (60) in der Dichtungsnut (235) aufgenommen ist, wobei die Dichtungsnut (235) und der Dichtungsring (60) den Dichtungsmechanismus bilden.

10. Die integrierte Ablationsnadel (100, 100b, 100c) nach Anspruch 1, die ferner einen Griff (30) umfasst, der mit einem proximalen Ende der Hülse (10) und einem proximalen Ende der Elektrodennadel (20) verbunden ist, wobei der Griff (30) dazu dient, die Hülse (10) dazu zu bringen, sich axial relativ zur Nadelstange (23) zu bewegen.

11. Die integrierte Ablationsnadel (100, 100b, 100c) nach einem der Ansprüche 1 bis 10, die ferner ein die Hülse (10) umgebendes Außenrohr (70) umfasst, wobei die Elektrodennadel (20) und die Hülse (10) relativ zum Außenrohr (70) axial beweglich sind.

12. Die integrierte Ablationsnadel (100, 100b, 100c) nach Anspruch 11, wobei das proximale Ende der Nadelspitze (21) mit einer Ausweichnut (237) um ihren Umfang herum versehen ist und wenn die Hülse (10) axial relativ zur Elektrodennadel (20) positioniert ist, der Schneidabschnitt (11) der Hülse (10) in der Ausweichnut (237) aufgenommen wird, und der Außendurchmesser der Hülse (10) gleich oder kleiner als der Durchmesser des proximalen Endes der Nadelspitze (21) ist.

13. Die integrierte Ablationsnadel (100, 100b, 100c) nach Anspruch 1, wobei der Probenentnahmeschlitz (231) eine lange, schmale Nut ist, die sich entlang einer axialen Richtung des Nadelschafts erstreckt; oder der Probenentnahmeschlitz (231) eine bogenförmige Nut ist, die sich in einer Umfangsrichtung des Nadelschafts erstreckt; oder der Probenentnahmeschlitz (231) eine ringförmige Nut ist, die sich einmal in einer Umfangsrichtung des Nadelschafts (23) erstreckt.

## Revendications

1. Aiguille d'ablation intégrée (100, 100b, 100c), comprenant un manchon (10) et une aiguille d'électrode (20) insérée de manière mobile dans le manchon (10), où l'aiguille d'électrode (20) comprend une pointe d'aiguille (21) à une extrémité distale de celle-ci et une tige d'aiguille (23) reliée à une extrémité proximale de la pointe d'aiguille (21), et où une fente de prélèvement (231) est ouverte au moins au niveau d'une partie de la tige d'aiguille (23) adjacente à la pointe d'aiguille (21), et où une partie de découpe (11) est prévue à une extrémité distale du manchon (10) ; et
où le manchon (10) est déplaçable axialement par rapport à la tige d'aiguille (23) pour exposer ou couvrir la fente de prélèvement (231) ; et où lorsque la fente de prélèvement (231) est exposée, un tissu autour de la tige d'aiguille (23) entre dans la fente de prélèvement (231), et lorsque la fente de prélèvement (231) est couverte, la partie de découpe (11) découpe le tissu à l'intérieur de la fente de prélèvement (231) pour le séparer du tissu à l'extérieur de la fente de prélèvement (231), en obtenant ainsi le tissu dans la fente de prélèvement (231) comme échantillon de biopsie ;
où l'aiguille d'électrode (20) est dotée d'un canal de refroidissement (233) s'étendant axialement depuis une face d'extrémité proximale de celle-ci jusqu'à un intérieur de la pointe d'aiguille (21), et un diamètre extérieur de la tige d'aiguille (23) est inférieur à un diamètre intérieur du manchon (20), et
**caractérisée en ce que**
le canal de refroidissement (233) communique avec la fente de prélèvement (231) et
une extrémité proximale du manchon (20) et une extrémité proximale de l'aiguille d'électrode (20) sont toutes deux reliées à un dispositif sous vide (40).

2. Aiguille d'ablation intégrée (100, 100b, 100c) selon la revendication 1, dans laquelle le manchon (10) est au moins en partie isolé.

3. Aiguille d'ablation intégrée (100, 100b, 100c) selon la revendication 1, dans laquelle la fente de prélèvement (231) inclut une pluralité de longues rainures étroites s'étendant le long d'une direction axiale de la tige d'aiguille, et où la pluralité de longues rainures étroites sont espacées les unes des autres le long d'une direction circonférentielle ou axiale de la tige d'aiguille ; ou la fente de prélèvement (231) inclut une pluralité de rainures en arc s'étendant dans une direction circonférentielle de la tige d'aiguille, et où la pluralité de rainures en arc sont espacées les unes des autres le long de la direction circonférentielle ou axiale de la tige d'aiguille (23) ; ou la fente de prélèvement (231) inclut une pluralité de rainures annulaires s'étendant sur un tour dans une direction circonférentielle de la tige d'aiguille (23), et où la pluralité de rainures annulaires sont espacées les unes des autres le long d'une direction axiale de la tige d'aiguille (23).

4. Aiguille d'ablation intégrée (100, 100b, 100c) selon la revendication 1, dans laquelle la partie de découpe (11) comprend une surface de découpe (113) inclinée par rapport à un axe du manchon (10), la surface de découpe (113) est plus proche de l'axe du manchon (10) en allant d'une extrémité proximale vers une extrémité distale de celle-ci, un premier angle (α) est formé entre une droite de tangente de la surface de découpe (113) et l'axe du manchon (10), et le premier angle (α) va de 9 degrés à 17 degrés.

5. Aiguille d'ablation intégrée (100, 100b, 100c) selon la revendication 4, dans laquelle un plan où l'extrémité distale de la surface de découpe (113) est située est perpendiculaire à l'axe du manchon (10) ; ou un second angle (β) est formé entre le plan où l'extrémité distale de la surface de découpe (113b) est située et l'axe du manchon (10b), où le second angle (β) va de 25 degrés à 35 degrés.

6. Aiguille d'ablation intégrée (100, 100b, 100c) selon la revendication 1, dans laquelle le manchon (10) est doté d'une première partie de positionnement, l'aiguille d'électrode (20) est dotée d'une seconde partie de positionnement, où la première partie de positionnement et la seconde partie de positionnement sont engageables l'une avec l'autre pour obtenir un positionnement axial entre l'aiguille d'électrode et le manchon.

7. Aiguille d'ablation intégrée (100, 100b, 100c) selon la revendication 6, dans laquelle le manchon (10) présente une rainure de réception circulaire (13) dans une paroi intérieure distale de celui-ci, et un premier épaulement annulaire (14) est formé à une jonction entre une lumière du manchon (10) et la rainure de réception (13) en tant que première partie de positionnement ; un diamètre de l'extrémité proximale de la pointe d'aiguille (21) est plus important qu'un diamètre de la tige d'aiguille (23), et un second épaulement annulaire (25) est formé à une jonction entre l'extrémité proximale de la pointe d'aiguille (21) et la tige d'aiguille (23) en tant que seconde partie de positionnement ; et lorsque le premier épaulement annulaire (14) bute contre le second épaulement annulaire (25), l'aiguille d'électrode (20) et le manchon (10) sont axialement positionnés, et une partie proximale de la pointe d'aiguille (21) est reçue dans la rainure de réception (13).

8. Aiguille d'ablation intégrée (100, 100b, 100c) selon la revendication 1, dans laquelle un mécanisme d'étanchéité est prévu sur le manchon (10) et/ou la tige d'aiguille (23) pour assurer l'étanchéité d'un espace radial entre le manchon (10) et la tige d'aiguille (23), et le mécanisme d'étanchéité est situé entre la fente de prélèvement (231) et la pointe d'aiguille (21) lorsque la fente de prélèvement (231) est couverte par le manchon (10).

9. Aiguille d'ablation intégrée (100, 100b, 100c) selon la revendication 8, dans laquelle la tige d'aiguille (23) est dotée d'au moins une rainure d'étanchéité circulaire (235) adjacente à la pointe d'aiguille (21), la rainure d'étanchéité (235) est située entre la fente de prélèvement (231) et la pointe d'aiguille (21), et une bague d'étanchéité (60) est reçue dans la rainure d'étanchéité (235), où la rainure d'étanchéité (235) et la bague d'étanchéité (60) forment le mécanisme d'étanchéité.

10. Aiguille d'ablation intégrée (100, 100b, 100c) selon la revendication 1, comprenant en outre une poignée (30) reliée à une extrémité proximale du manchon (10) et à une extrémité proximale de l'aiguille d'électrode (20), la poignée (30) est utilisée pour amener le manchon (10) à se déplacer axialement par rapport à la tige d'aiguille (23).

11. Aiguille d'ablation intégrée (100, 100b, 100c) selon l'une quelconque des revendications 1 à 10, comprenant en outre un tube extérieur (70) entourant le manchon (10), et l'aiguille d'électrode (20) et le manchon (10) sont axialement déplaçables par rapport au tube extérieur (70).

12. Aiguille d'ablation intégrée (100, 100b, 100c) selon la revendication 11, dans laquelle l'extrémité proximale de la pointe d'aiguille (21) est dotée d'une rainure de garde (237) autour d'une circonférence de celle-ci, et lorsque le manchon (10) est axialement positionné par rapport à l'aiguille d'électrode (20), la partie de découpe (11) du manchon (10) est reçue dans la rainure de garde (237), et un diamètre extérieur du manchon (10) est inférieur ou égal à un diamètre de l'extrémité proximale de la pointe d'aiguille (21).

13. Aiguille d'ablation intégrée (100, 100b, 100c) selon la revendication 1, dans laquelle la fente de prélèvement (231) est une longue rainure étroite s'étendant le long d'une direction axiale de la tige d'aiguille ; ou la fente de prélèvement (231) est une rainure en arc s'étendant dans une direction circonférentielle de la tige d'aiguille ; ou la fente de prélèvement (231) est une rainure annulaire s'étendant sur un tour dans une direction circonférentielle de la tige d'aiguille (23).
